# EUROPÄISCHE PATENTSCHRIFT

(11) **EP 3 431 047 B1**
(45) Veröffentlichungstag und Bekanntmachung des Hinweises auf die Patenterteilung: **15.04.2020**
(21) Anmeldenummer: 18183885.5
(22) Anmeldetag: 17.07.2018
(51) Int. Cl.: A61F 2/52

(54) **VOLUMENANPASSBARE BRUSTPROTHESE**
VOLUME-ADAPTABLE BREAST PROSTHESIS
PROTHÈSE MAMMAIRE ADAPTABLE AU VOLUME

(30) Priorität: 20.07.2017 DE 102017116397
(43) Veröffentlichungstag der Anmeldung: 23.01.2019
(73) Patentinhaber: Amoena Medizin-Orthopädie-Technik GmbH, 83064 Raubling (DE)
(72) Erfinder: Stelter, Nils, 83112 Frasdorf (DE); Wild, Helmut, 83071 Stephanskirchen (DE)
(74) Vertreter: Laufhütte, Dieter

(56) Entgegenhaltungen:
- WO-A1-2017/080664
- DE-A1- 4 115 428
- DE-B1- 2 457 041
- US-A1- 2013 131 798

## Beschreibung

Die Erfindung betrifft eine volumenanpassbare Brustprothese mit einem elastisch verformbaren Körper, dessen Außenoberfläche der Brustform nachgebildet ist.

Brustprothesen werden nach typischerweise krebsbedingten (Teil)Entfernungen der Brust aus kosmetischen Gründen eingesetzt. Neben einer der natürlichen Brust möglichst nahekommenden Form und Haptik soll insbesondere ein hoher Tragekomfort erreicht werden.

Im Stand der Technik ist es bekannt, die Innenseite der Prothese mit einer plastisch verformbaren Schichte zu versehen, die sich der Form des Narbengewebes anpasst. Bei diesen bekannten Prothesen kann jedoch das Problem auftreten, dass durch diese plastisch verformbare Schicht das Narbengewebe okklusiv abgeschlossen wurde. Dies kann zu Hitzestaus und Schweißbildung unter der Prothese führen, was dem Tragekomfort abträglich ist und auch zu Hautreaktionen führen kann. Vor diesem Hintergrund wird in der WO 2017/080664 A1 eine Brustprothese vorgestellt, die einen plastisch verformbaren inneren Körper aufweist, dessen Innenoberfläche sich an Unebenheiten des Gewebes anpassen kann, wobei die Innenoberfläche des inneren Körpers eine dreidimensionale Struktur aufweist und/oder mit einem Textil versehen ist, die bzw. das so ausgeführt ist, dass eine Belüftung der Gewebeoberfläche erreicht werden kann. So kann sich die Innenseite der Brustprothese an die Form des Narbengewebes anpassen, ohne einen Luftaustausch zu verhindern.

Die Druckschrift DE4115428 A1 offenbart eine aufblasbare Brustprothese, welche in ihrer Größe an einen verbleibende Brust angepasst werden kann.

Die Brustprothese der WO 2017/080664 A1 hat jedoch den Nachteil, dass sie sich zwar in gewissem Umfang an die Form des Narbengewebes anpassen kann, dass eine Anpassung an die Größe der verbleibenden Brust der Trägerin nicht und eine vollständige Anpassung an die Größe der unterhalb der Prothese liegenden Gewebetopographie oftmals nicht erfolgen kann.

Aufgabe der Erfindung ist es, eine Brustprothese bereitzustellen, deren Innenseite der Brustprothese an die Form des Narbengewebes anpassen, ohne einen Luftaustausch zu verhindern, und die darüber hinaus in ihrer Größe individuell an eine Patientin angepasst werden kann.

Vor diesem Hintergrund betrifft die Erfindung eine Brustprothese mit einem elastisch verformbaren äußeren Körper, dessen Außenoberfläche der Brustform nachgebildet ist, und mit einem plastisch verformbaren inneren Körper, dessen Innenoberfläche sich an Unebenheiten des Gewebes anpassen kann, wobei die Innenoberfläche des inneren Körpers eine dreidimensionale Struktur aufweist und/oder mit einem Textil versehen ist, die bzw. das so ausgeführt ist, dass eine Belüftung der Gewebeoberfläche erreicht werden kann. Erfindungsgemäß ist vorgesehen, dass zwischen dem äußeren Körper und dem inneren Körper ein Zwischenkörper mit einer Zwischenkammer angeordnet ist, der so ausgebildet ist, dass sein Volumen durch Befüllen mit einem Fluid angepasst werden kann.

In einer Ausführungsform liegen der äußere Körper, der Zwischenkörper und der innere Körper direkt aneinander an.

In einer Ausführungsform besteht die Brustprothese aus dem äußeren Körper, dem Zwischenkörper, dem inneren Körper und der Struktur bzw. dem Textil.

In einer Ausführungsform ist der äußere Körper schalenförmig. Auch der Zwischenkörper und der innere Körper können schalenförmig sein.

In einer Ausführungsform besteht die Brustprothese aus dem äußeren Körper, dem Zwischenkörper, dem inneren Körper und der Struktur bzw. dem Textil.

In einer Ausführungsform ist der äußere Körper schalenförmig. Auch der Zwischenkörper und der innere Körper können schalenförmig sein.

In einer Ausführungsform weist der äußere Körper eine in einer äußeren Kammer eingeschlossene gummielastische Masse auf. Die äußere Kammer kann beispielsweise durch einen Folienbeutel gebildet werden. Bei der gummielastischen Masse kann es sich beispielsweise um eine Zweikomponenten-Silikonkautschuk-Masse handeln.

In einer Ausführungsform weist der innere Körper eine in einer inneren Kammer eingeschlossene plastisch verformbare Masse auf. Auch die innere Kammer kann beispielsweise durch einen Folienbeutel gebildet werden.

Bei der plastisch verformbaren Masse kann es sich um eine homogene oder heterogene Masse handeln. Die Masse kann thixotrope Eigenschaften aufweisen. Bei der Masse kann es sich um eine strukturviskose Masse mit Fließgrenze handeln. Beispiele umfassen geringvernetzte Silikongele, vorzugsweise solche mit wenigstens zwei Komponenten, welche gegebenenfalls ferner ein oder mehrere Additive enthalten können. Geeignete Additive umfassen Phasenwechselmaterialien, expandierte Leichtfüllstoffe, beispielsweise poröse oder gasgefüllte Partikel (im Rahmen des Herstellungsprozesses können unexpandierte Leichtfüllstoffe zugegeben werden, die durch Temperatur während des Herstellungsprozesses expandieren), Verdickungsmittel, Aerosile oder Thixotropierungsmittel. Beispiele weiterer geeigneter plastisch verformbarer Massen sind in der EP 0 768 068 A1, der EP 2 133 042 A1 und der WO 2014/078021 A1 offenbart.

In einer Ausführungsform ist vorgesehen, dass der Zwischenkörper durch einen Folienbeutel gebildet wird, dass also die Zwischenkammer durch einen Folienbeutel begrenzt wird.

In einer Ausführungsform werden ein oder mehrere der Folienbeutel durch zwei entlang des gemeinsamen Umfangs verschweißte Kunststofffolien gebildet.

Es kann vorgesehen sein, dass die den äußeren Körper, den Zwischenkörper und den inneren Körper bildenden Folienbeutel durch vier entlang des gemeinsamen Umfangs verschweißte Kunststofffolien gebildet werden. Dann entsprechen die innere Begrenzungsfolie des äußeren Körpers der äußeren Begrenzungsfolie des Zwischenkörpers und die innere Begrenzungsfolie des Zwischenkörpers der äußeren Begrenzungsfolie inneren Körpers.

In einer Ausführungsform ist eine verschließbare Durchgangsöffnung vorgesehen, die von der Zwischenkammer an die Außenseite der Brustprothese reicht. Anhand dieser Durchgangsöffnung kann die Befüllung der Zwischenkammer mit einem Fluid vorgenommen werden. Als Füllmaterialien eignen sich Flüssigkeiten oder Gele, insbesondere Silikongele. In einem nichterfindungsgemäßen Beispiel kann das Füllmaterial auch Gase umfassen. Bei der Durchgangsöffnung kann es sich um einen Kanal oder einen Durchbruch handeln.

In einer Ausführungsform ist vorgesehen, dass sich die Durchgangsöffnung an der Seitenkontur der Brustprothese befindet. Beispielsweise kann vorgesehen sein, dass es sich bei der Durchgangsöffnung um einen Kanal handelt, der zwischen zwei Lagen der Schweißnaht zwischen den beiden Folien verläuft, welche den Zwischenkörper bilden.

In einer Ausführungsform ist vorgesehen, dass sich die Durchgangsöffnung an der Innenoberfläche der Brustprothese befindet.

Die Brustprothese kann in der Draufsicht eine etwa tropfenförmige Gestalt haben, die der Form der Brust nachgebildet ist. In diesem Fall kann vorgesehen sein, dass die Durchgangsöffnung sich an oder im Bereich von einem der beiden Schenkel befindet. Dies hat den Vorteil, dass die Prothese in eine BH-Schale eingelegt und dann sternumseitig oder achselseitig befüllt werden kann.

In einer Ausführungsform ist vorgesehen, dass in der Durchgangsöffnung ein Ventil vorgesehen ist. Geeignet sind selbstdichtende Ventile wie beispielsweise ein Flatterventil oder ein Duckbill-Ventil. Für den Tragekomfort ist von Vorteil, wenn das Ventil flexibel bzw. elastisch ist.

Die dreidimensionale Struktur bzw. das Textil ist vorzugsweise plastisch und/oder elastisch verformbar. Sie bzw. ist so geformt, dass eine Belüftung der Haut gewährleistet wird.

In einer Ausführungsform ist die dreidimensionale Struktur an die Innenoberfläche des inneren Körpers angeformt. In einer anderen Ausführungsform ist die dreidimensionale Struktur in die Innenoberfläche des inneren Körpers eingearbeitet. Beispielsweise kann vorgesehen sein, dass die dreidimensionale Struktur einen integralen Teil der Kunststofffolie bildet, welche die innere Begrenzung des inneren Körpers darstellt. Sie kann an dieser Folie angeformt bzw. in diese eingearbeitet sein.

In einer Ausführungsform ist die dreidimensionale Struktur auf die Innenoberfläche des inneren Körpers aufgeklebt oder aufgeschweißt.

In einer Ausführungsform handelt es sich bei der dreidimensionalen Struktur um einer Struktur aus Kunststoff. Bei dem Kunststoffmaterial kann es sich um dasselbe Material handeln, dass für die Kunststofffolie verwendet wird, welche die innere Begrenzung des inneren Körpers darstellt. Alternativ können für die Folie und die Struktur unterschiedliche Kunststoffmaterialien verwendet werden.

In einer Ausführungsform umfasst die dreidimensionale Struktur Kanäle, Stege und/oder Noppen.

In einer Ausführungsform ist das Textil auf die Innenoberfläche des inneren Körpers aufgeklebt oder aufgeschweißt.

In einer anderen Ausführungsform ist das Textil anhand eines Haltemittels an der Innenoberfläche des inneren Körpers angebracht. Beispielsweise kann vorgesehen sein, dass das Textil anhand einer auf die Innenoberfläche des inneren Körpers aufgeklebten oder aufgeschweißten Klettfläche an der Innenoberfläche des inneren Körpers angebracht ist.

In einer Ausführungsform handelt es sich bei dem Textil um ein dreidimensionales Textil mit einer Stärke von beispielsweise größer 2 mm und vorzugsweise von größer 5 mm. Beispiele umfassen zwei- oder mehrflächige Textile wie beispielsweise Abstandsgewirke oder Abstandsgestricke.

In einer Ausführungsform weist das Textil zumindest an der dem Körper zugewandten Oberfläche eine Oberflächenstruktur wie beispielsweise Rippen oder Noppen auf.

In einer Ausführungsform weist das Textil Naturfasern auf oder besteht daraus. Naturfasern können Vorteile in der Hautverträglichkeit und in einer schwachen Geruchsannahme haben. Beispiele geeigneter Naturfasern umfassen Baumwollfasern oder Wollfasern.

In einer Ausführungsform weist das Textil Kunstfasern auf oder besteht daraus. Kunstfasern können Vorteile in einer guten Schweißableitung und einer geringen Oberflächenrauheit haben.

Auch Kombinationen aus Natur- und Kunstfasern sind denkbar und von der Erfindung umfasst.

Die Erfindung betrifft ferner ein Verfahren zur Anpassung einer erfindungsgemäßen Brustprothese, wobei das Volumen der Brustprothese durch Befüllen der Zwischenkammer angepasst wird. Die Befüllung kann beispielsweise erfolgen, während die Brustprothese in einer BH-Schale aufgenommen und von der Patientin getragen wird. Die Befüllung kann beispielsweise sternumseitig oder achselseitig erfolgen. Als Füllmaterialien eignen sich Flüssigkeiten oder Gele, insbesondere Silikongele. Die Befüllung erfolgt vorzugsweise durch Anlegen einer Befüllvorrichtung an die Außenseite der Durchgangsöffnung. Als Befüllvorrichtungen eignen sich beispielweise manuelle oder elektrische Pumpvorrichtungen.

Weitere Einzelheiten und Vorteile ergeben sich aus dem nachfolgend anhand der Figuren diskutierten Ausführungsbeispiel. In den Figuren zeigen:

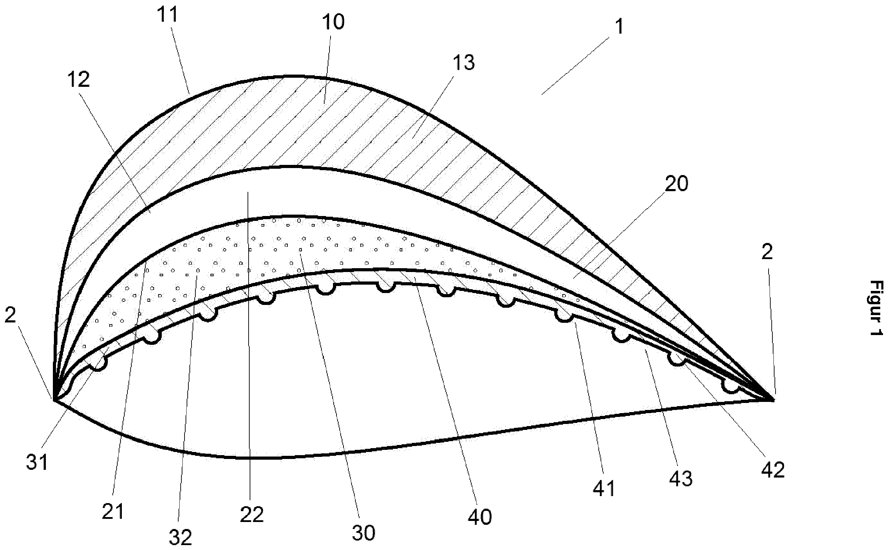

- Figur 1:: eine Schnittansicht einer erfindungsgemäßen Brustprothese; und

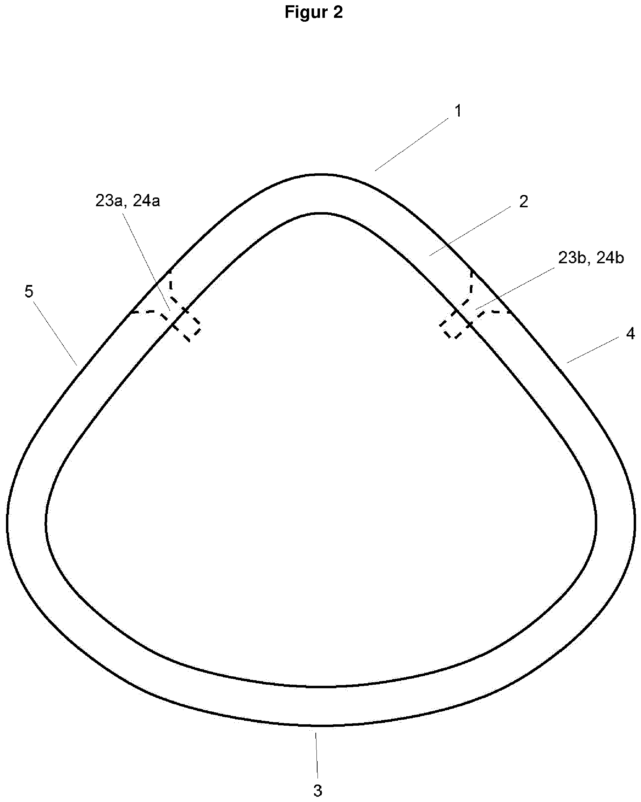

- Figur 2:: eine Draufsicht auf eine erfindungsgemäße Brustprothese.

In den Figuren ist eine Ausführungsform einer erfindungsgemäßen Brustprothese gezeigt, die allgemein mit dem Bezugszeichen 1 bezeichnet ist.

Die Außenseite der Brustprothese wird durch einen schalenförmigen äußeren Körper 10 gebildet, der elastisch verformbar ist. Die Innenseite der Brustprothese wird durch einen ebenfalls schalenförmigen inneren Körper 30 gebildet. Die Körper 10 und 30 sind deckungsgleich, d.h., haben in der Draufsicht dieselbe Fläche und Form. Zwischen dem äußeren Körper 10 und dem inneren Körper 30 ist vollflächig ein expandierbarer Zwischenkörper 20 angeordnet, der ebenfalls schalenförmig ist.

Der äußere Körper 10 besteht aus zwei entlang dem Umfangsrand verschweißten Kunststofffolien, nämlich einer äußeren Folie 11 und einer inneren Folie 12, die eine Kammer 13 bilden, in der sich eine gummielastische Zweikomponenten-Silikonkautschuk-Masse befindet.

Der Zwischenkörper 20 wird durch Aufschweißen einer weiteren Kunststofffolie 21 an die Innenseite des äußeren Körpers 10 gebildet.

Der innere Körper 30 wird durch Aufschweißen einer wiederum weiteren Kunststofffolie 31 an die Innenseite des Zwischenkörpers 20 und das Füllen der zwischenliegenden Kammer 32 mit einem Füllmaterial gebildet. Bei dem Füllmaterial handelt es sich um eine plastisch verformbare Masse mit thixotropen Materialeigenschaften, bei der es sich um ein gering vernetztes Silikongel zweier unterschiedlicher Silikonkomponenten handelt, das ferner Phasenwechselmaterial, expandierte Leichtfüllstoffe, Verdickungsmittel, Aerosole und Thixotropierungsmittel enthält.

Die Kunststofffolien 11, 12, 21 und 31 sind allesamt aus identischem Material gefertigt. Sie sind entlang einer gemeinsamen Schweißnaht 2 im Randbereich der Prothese 1 verbunden, wobei diese nicht unbedingt am äußeren Rand liegen muß, sondern um beispielsweise 10mm - 20mm umlaufend nach innen versetzt sein kann.

Auf der Innenfolie 31 des plastisch verformbaren inneren Körpers 30 ist ein strukturiertes Kunststoff-Inlay 40 aufgeklebt. Die Klebeoberfläche des Inlays 40 ist glatt, um eine gute Haftung an der Innenfolie 31 des inneren Körpers 30 zu gewährleisten. Die dem inneren Körper 30 abgewandte Oberfläche des Inlays 40 weist eine dreidimensionale Strukturierung 41 auf, bei der es sich um eine regelmäßige Anordnung von Noppen 42 und Belüftungs- bzw. Faltkanälen 43 handelt.

Im Inneren des Zwischenkörpers 20 ist eine Zwischenkammer 22 gebildet, die mit einem Fluid gefüllt werden kann. Je nach dem Ausmaß der Befüllung nimmt die Dicke des Zwischenkörpers um ein bestimmtes Maß zu. So kann das Volumen der Brustprothese 1 in Anbetracht der Größe der verbleibenden Brust und des Verlaufs des Narbengewebes angepasst werden.

Die Brustprothese hat in der Draufsicht eine insgesamt tropfenförmige Gestalt, wobei die Unterseite durch die Rundung 3 und die beiden zusammenlaufenden Seiten durch linke und rechte Schenken 4 und 5 gebildet werden.

Zur Befüllung des Zwischenkörpers 20 sind zwei Durchgangskanäle 23a und 23b mit je einem elastischen Flatterventil 24a und 24b vorgesehen. Die Kanäle 23 sind seitlich an der Brustprothese angeordnet und liegen zwischen den Folien 12 und 21, also innerhalb der Schweißnaht 2. Sie stehen ausgehend von der Zwischenkammer 20 radial nach außen. Sie sind an den beiden Schenkeln 4 und 5 der Brustprothese 1 angeordnet, sodass die Befüllung nach Einlegen der Prothese in eine Schale des von der Patientin getragenen BHs wahlweise sternumseitig oder achselseitig befüllt werden kann, unabhängig davon, ob die Prothese links oder rechts getragen wird.

Im Rahmen der Befüllung der Zwischenkammer 22 wird durch eine Pumpe eine Flüssigkeit oder ein Gel, vorzugsweise ein Silikongel von der Außenseite durch den Kanal 23 und das selbstschließende Flatterventil 24 gedrückt.

## Patentansprüche

1. Brustprothese (1) mit einem elastisch verformbaren äußeren Körper (10), dessen Außenoberfläche der Brustform nachgebildet ist, und mit einem plastisch verformbaren inneren Körper (30), dessen Innenoberfläche sich an Unebenheiten des Gewebes anpassen kann, wobei die Innenoberfläche des inneren Körpers (30) eine dreidimensionale Struktur (41) aufweist und/oder mit einem Textil versehen ist, die bzw. das so ausgeführt ist, dass eine Belüftung der Gewebeoberfläche erreicht werden kann,
**dadurch gekennzeichnet,**
**dass** zwischen dem äußeren Körper (10) und dem inneren Körper (30) ein Zwischenkörper (20) mit einer Zwischenkammer (22) angeordnet ist, der so ausgebildet ist, dass sein Volumen durch Befüllen mit einer Flüssigkeit oder einem Gel angepasst werden kann.

2. Brustprothese nach Anspruch 1, **dadurch gekennzeichnet, dass** der äußere Körper (10) eine in einer durch einen Folienbeutel (11, 12) gebildeten äußeren Kammer (13) eingeschlossene gummielastische Masse, vorzugsweise eine Zweikomponenten-Silikonkautschuk-Masse aufweist.

3. Brustprothese nach einem der vorhergehenden Ansprüche, **dadurch gekennzeichnet, dass** der innere Körper (30) eine in einer vorzugsweise durch einen Folienbeutel (21, 31) gebildeten inneren Kammer (32) eingeschlossene plastisch verformbare Masse aufweist.

4. Brustprothese nach einem der vorhergehenden Ansprüche, **dadurch gekennzeichnet, dass** der Zwischenkörper (20) durch einen Folienbeutel (12, 21) gebildet wird.

5. Brustprothese nach einem der vorhergehenden Ansprüche, **dadurch gekennzeichnet, dass** eine verschließbare Durchgangsöffnung (23) vorgesehen ist, die von der Zwischenkammer (22) an die Außenseite der Brustprothese (1) reicht, wobei vorzugsweise vorgesehen ist, dass in der Durchgangsöffnung (23) ein vorzugsweise selbstdichtendes Ventil (24) vorgesehen ist.

6. Brustprothese nach Anspruch 5, **dadurch gekennzeichnet, dass** sich die Durchgangsöffnung (23) an der Seitenkontur (3, 4, 5) oder der Innenoberfläche der Brustprothese befindet.

7. Brustprothese nach einem der vorhergehenden Ansprüche, **dadurch gekennzeichnet, dass** die dreidimensionale Struktur (41) an die Innenoberfläche des inneren Körpers (30) angeformt ist oder in diese eingearbeitet ist oder dass die dreidimensionale Struktur auf die Innenoberfläche des inneren Körpers (30) aufgeklebt oder aufgeschweißt ist.

8. Brustprothese nach einem der vorhergehenden Ansprüche, **dadurch gekennzeichnet, dass** es sich bei der dreidimensionalen Struktur (41) um eine Struktur aus Kunststoff handelt und/oder dass die dreidimensionale Struktur als Strukturelemente Kanäle (43), Stege und/oder Noppen (42) umfasst.

9. Brustprothese nach einem der vorhergehenden Ansprüche, **dadurch gekennzeichnet, dass** das Textil auf die Innenoberfläche des inneren Körpers (30) aufgeklebt oder aufgeschweißt ist oder dass das Textil anhand eines Haltemittels an der Innenoberfläche des inneren Körpers (30) angebracht ist, beispielsweise anhand einer auf die Innenoberfläche des inneren Körpers (30) aufgeklebten oder aufgeschweißten Klettfläche.

10. Verfahren zur Anpassung einer Brustprothese (1) nach einem der vorhergehenden Ansprüche,
**dadurch gekennzeichnet,**
**dass** das Volumen der Brustprothese (1) durch Befüllen der Zwischenkammer (22) mit einer Flüssigkeit oder einem Gel angepasst wird.

## Claims

1. Breast prosthesis (1) having an elastically deformable outer body (10) whose outer surface is patterned on the shape of the breast and having a plastically deformable inner body (30) whose inner surface can adapt to irregularities of the tissue, wherein the inner surface of the inner body (30) has a three-dimensional structure (41) and/or is provided with a textile that is respectively designed such that a ventilation of the tissue surface can be achieved,
**characterized in that**
an intermediate body (20) having an intermediate chamber (22) is arranged between the outer body (10) and the inner body (30) and is configured such that its volume can be adapted by filling with a fluid or a gel.

2. Breast prosthesis in accordance with claim 1, **characterized in that** the outer body (10) has a rubber elastic compound, preferably a two-component silicone rubber compound, enclosed in an outer chamber (13) formed by a film bag (11, 12).

3. Breast prosthesis in accordance with one of the preceding claims, **characterized in that** the inner body (30) has a plastically deformable compound enclosed in an inner chamber (32) preferably formed by a film bag (21,31).

4. Breast prosthesis in accordance with one of the preceding claims, **characterized in that** the intermediate body (20) is formed by a film bag (12, 21).

5. Breast prosthesis in accordance with one of the preceding claims, **characterized in that** a closable passage opening (23) is provided that extends from the intermediate chamber (22) to the outer side of the breast prosthesis (1), wherein provision is preferably made that a preferably self-closing valve (24) is provided in the passage opening (23).

6. Breast prosthesis in accordance with claim 5, **characterized in that** the passage opening (23) is located at the side contour (3, 4, 5) or the inner surface of the breast prosthesis.

7. Breast prosthesis in accordance with one of the preceding claims, **characterized in that** the three-dimensional structure (41) is shaped to the inner surface of the inner body (30) or is worked into it, or **in that** the three-dimensional structure is adhesively bonded or welded to the inner surface of the inner body (30).

8. Breast prosthesis in accordance with one of the preceding claims, **characterized in that** the three-dimensional structure (41) is a structure of plastic, and/or **in that** the three-dimensional structure comprises channels (43), webs and/or nubs (42) as structural elements.

9. Breast prosthesis in accordance with one of the preceding claims, **characterized in that** the textile is adhesively bonded or welded to the inner surface of the inner body (30), or **in that** the textile is attached to the inner surface of the inner body (30) using a holding means, for example using a hook and loop surface adhesively bonded or welded to the inner surface of the inner body (30).

10. Method of adapting a breast prosthesis (1) in accordance with one of the preceding claims,
**characterized in that**
the volume of the breast prosthesis (1) is adapted by filling the intermediate chamber (22) with a fluid or a gel.

## Revendications

1. Prothèse mammaire (1) comprenant un corps extérieur (10), susceptible de subir une déformation élastique, dont la surface extérieure reproduit la forme du sein, et comprenant un corps intérieur (30), susceptible de subir une déformation plastique, dont la surface intérieure peut s'adapter à des irrégularités du tissu, la surface intérieure du corps intérieur (30) comportant une structure tridimensionnelle (41) et/ou étant pourvue d'un textile, qui est réalisé(e) de telle manière qu'une ventilation de la surface du tissu peut être obtenue,
**caractérisée en ce que**
entre le corps extérieur (10) et le corps intérieur (30), se trouve un corps intermédiaire (20) doté d'une chambre intermédiaire (22), qui est conçu de telle manière que son volume peut être adapté par remplissage avec un liquide ou un gel.

2. Prothèse mammaire selon la revendication 1, **caractérisée en ce que** le corps extérieur (10) comporte une masse élastique caoutchouteuse, de préférence une masse de caoutchouc silicone à deux composants, enfermée dans une chambre (13) extérieure formée par un sachet en film (11, 12).

3. Prothèse mammaire selon l'une des revendications précédentes, **caractérisée en ce que** le corps intérieur (30) comporte une masse susceptible de subir une déformation plastique, enfermée dans une chambre (32) intérieure de préférence formée par un sachet en film (21, 31).

4. Prothèse mammaire selon l'une des revendications précédentes, **caractérisée en ce que** le corps intermédiaire (20) est formé par un sachet en film (12, 21).

5. Prothèse mammaire selon l'une des revendications précédentes, **caractérisée en ce qu'**un orifice de passage (23) susceptible d'être fermé est prévu, qui part de la chambre intermédiaire (22) pour atteindre le côté extérieur de la prothèse mammaire (1), une soupape (24) de préférence autoétanchéifiante étant de préférence prévue dans l'orifice de passage (23).

6. Prothèse mammaire selon la revendication 5, **caractérisée en ce que** l'orifice de passage (23) se trouve sur le contour latéral (3, 4, 5) ou sur la surface intérieure de la prothèse mammaire.

7. Prothèse mammaire selon l'une des revendications précédentes, **caractérisée en ce que** la structure tridimensionnelle (41) est formée sur la surface intérieure du corps intérieur (30) ou est incorporée dans celle-ci ou **en ce que** la structure tridimensionnelle est collée ou soudée sur la surface intérieure du corps intérieur (30).

8. Prothèse mammaire selon l'une des revendications précédentes, **caractérisée en ce que** la structure tridimensionnelle (41) est une structure en matière plastique et/ou **en ce que** la structure tridimensionnelle comprend des canaux (43), des nervures et/ou des protubérances (42) en guise d'éléments de structure.

9. Prothèse mammaire selon l'une des revendications précédentes, **caractérisée en ce que** le textile est collé ou soudé sur la surface intérieure du corps intérieur (30) ou **en ce que** le textile est appliqué sur la surface intérieure du corps intérieur (30) à l'aide d'un moyen de retenue, par exemple à l'aide d'une surface agrippante collée ou soudée sur la surface intérieure du corps intérieur (30).

10. Procédé d'adaptation d'une prothèse mammaire (1) selon l'une des revendications précédentes,
**caractérisé en ce que**
le volume de la prothèse mammaire (1) est adapté en remplissant la chambre intermédiaire (22) avec un liquide ou un gel.
